# FASCICULE DE BREVET EUROPEEN

(11) **EP 4 076 116 B1**
(45) Date de publication et mention de la délivrance du brevet: **01.10.2025**
(21) Numéro de dépôt: 20845164.1
(22) Date de dépôt: 14.12.2020
(51) Int. Cl.: A47K 7/08, A61H 19/00, A61H 35/00, A61M 3/02

(54) **DISPOSITIF SANITAIRE À MAIN AINSI QU'UN SACHET HYGIÉNIQUE POUR LE LAVAGE ET LE SÉCHAGE DES PARTIES INTIMES**
HANDSANITÄREINRICHTUNG UND KÖRPERHYGIENEBEUTEL ZUM WASCHEN UND TROCKNEN VON INTIMKÖRPERTEILEN
HAND-HELD SANITARY DEVICE AND PERSONAL-HYGIENE SACHET FOR WASHING AND DRYING INTIMATE BODY PARTS

(30) Priorité: 19.11.2020 FR 2011895; 16.12.2019 FR 1914477
(43) Date de publication de la demande: 26.10.2022
(73) Titulaire: Bernier, Gilbert, 37300 Joué-lès-Tours (FR)
(72) Inventeur: Bernier, Gilbert, 37300 Joué-lès-Tours (FR)
(86) Numéro de dépôt international: PCT/FR2020/000273
(87) Numéro de publication internationale: WO 2021/123515

(56) Documents cités:
- US-A1- 2007 000 041

## Description

Dispositif sanitaire à main ainsi qu'un sachet hygiénique pour le lavage et le séchage des parties intimes
La présente invention concerne un dispositif sanitaire à main ainsi qu'un sachet hygiénique à usage unique pour le lavage par de légers frottements et le séchage à l'air chaud des parties intimes.

On connait des dispositifs de lavage des parties intimes comme les éjecteurs d'eau installés dans des cuvettes de toilettes et les douchettes à main. L'inconvénient de ces dispositifs est la difficulté pour l'utilisateur d'éviter des éclaboussures en utilisant un jet d'eau suffisant pour atteindre sa partie intime qui doit être lavée, en particulier sa zone périanale après défécation. Le brevet US2007/000041 décrit un petit récipient pour liquide qui est exclusivement destiné au lavage vaginal. La présente invention permet de remédier à ces inconvénients en proposant un dispositif sanitaire à main qui permet l'utilisation d'un sachet hygiénique rempli d'eau et tendu pour le lavage superficiel des parties intimes par de légers frottements. Le dispositif est d'un maniement commode pour pouvoir frotter légèrement avec précision la partie intime qui doit être lavée, en particulier la zone périanale après défécation, sans mouiller les autres parties du corps ni éclaboussures. Il permet ainsi un lavage satisfaisant des parties intimes avec un faible écoulement d'eau et un court temps de séchage à l'air chaud. Il peut être utilisé en se servant des cuvettes de toilettes courantes et sans nécessité d'utilisation de papier de toi lette dont la fabrication est parmi les causes de la déforestation dans le monde. Un appareil pour le chauffage de l'eau et la génération d'un flux d'air chaud est prévu et peut être considéré comme un chauffe-eau instantané et séchoir.

Un récipient d'eau et une poire de lavage avec des filetages femelles qui permettent leur vissage sur le dispositif sanitaire pour des utilisations portables sont prévus.

Des modes de réalisation particuliers et avantageux de l'invention sont indiqués ci-dessous et décrits en détail plus loin.

Selon un mode de réalisation, le dispositif sanitaire permet l'utilisation d'un sachet hygiénique rempli d'eau et tendu pour le lavage superficiel des parties intimes par de légers frottements. Il comprend une butée conique avec un anneau qui est soumis à l'action d'un ressort de compression pour un maintien étanche d'un sachet hygiénique à usage unique, un robinet de contrôle de l'écoulement de l'eau, un filetage pour le raccordement d'un tuyau flexible d'alimentation en eau, un passage d'eau, une sortie d'eau et un anneau ouvert pour le maintien d'un tuyau flexible d'alimentation en air chaud. Le sachet hygiénique a une sortie d'eau qui est couverte avec une bande qui permet d'éviter des éclaboussures et une partie cylindrique avec une ouverture latérale qui permet d'orienter le flux d'air chaud vers les parties intimes en tenant commodément le dispositif sanitaire avec une main durant le séchage. Ce dispositif peut aussi être utilisé pour le lavage vaginal, la rééducation du périnée et le plaisir vaginal pour la femme en utilisant le sachet hygiénique prévu pour chaque usage.

Un autre mode de réalisation qui consiste en ce que le dispositif sanitaire comprend une extrémité lisse qui doit être couverte avec un sachet hygiénique imperméable pour être utilisée pour le frottement des parties intimes durant le lavage. Une pince qui maintient ce sachet est réalisée sur le corps du dispositif.

Selon encore un autre mode de réalisation, le dispositif sanitaire a une extrémité lisse pliable et il est facile à transporter durant le voyage. Il est destiné au lavage superficiel des parties intimes.

Les sachets hygiéniques prévus pour les divers usages selon l'invention sont fabriqués à partir d'un matériau imperméable, essentiellement biodégradable et compostable. Ils sont à usage unique et peuvent être commercialisés dans des sachets fermés d'un ou quelques sachets hygiéniques ou éventuellement sous forme de rouleaux de sachets hygiéniques prédécoupés. Ils peuvent être transparents ou opaques.

Le chauffe-eau instantané et séchoir comprend une résistance montée autour d'un conduit d'eau qui constitue la partie principale du chauffe-eau instantané, une deuxième résistance avec un petit ventilateur qui constitue la partie principale du séchoir, des protections contre le surchauffage, des interrupteurs, un réducteur de pression qui permet de limiter la pression de l'eau dans le tuyau flexible d'alimentation en eau et dans le sachet hygiénique qui doit être rempli d'eau et utilisé pour le frottement des parties intimes durant le lavage, un temporisateur qui permet de fixer la durée de fonctionnement du séchoir après chaque lavage, un pressostat avec un contact électrique inverseur qui permet la commutation de l'alimentation électrique entre le chauffe-eau instantané et le séchoir en ouvrant et en fermant le robinet de contrôle de l'écoulement de l'eau, une entrée d'eau, une sortie d'eau chaude, une entrée d'air et une sortie d'air chaud. Le contact inverseur du pressostat a un contact normalement ouvert, un contact normalement fermé et entre les deux un contact commun. Le temporisateur a deux bornes pour la connexion de son alimentation électrique et un contact normalement fermé qui s'ouvre après la durée préfixée. Cette durée est comptée à partir de sa mise sous tension.

D'autres caractéristiques et avantages de l'invention résulteront de la description ci-après d'exemples non limitatifs en référence aux dessins annexés dans lesquels :
La figure 1 représente une vue de profile d'un dispositif sanitaire avec un sachet hygiénique transparent qui peut être rempli d'eau et utilisé pour le lavage superficiel des parties intimes par de légers frottements.
La figure 2 représente une vue de face d'un sachet hygiénique destiné au lavage vaginal.
La figure 3 représente une vue de face d'un sachet hygiénique destiné à la rééducation du périnée et au plaisir vaginal pour la femme.
La figure 4 représente une vue de profile d'un dispositif sanitaire avec une extrémité lisse qui est couverte d'un sachet hygiénique transparent.
La figure 5 représente une vue de profile d'un dispositif sanitaire pliable et portable.

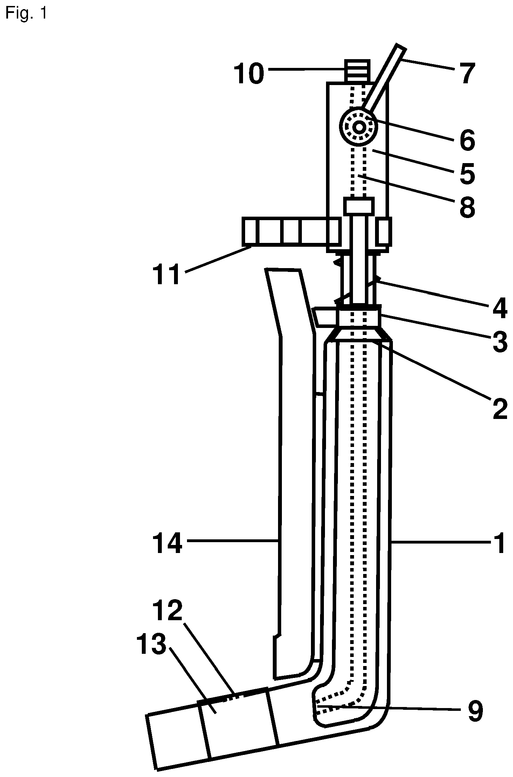

La figure 1 représente un dispositif sanitaire avec un sachet hygiénique (1) transparent. Le dispositif sanitaire comprend une butée conique (2) avec un premier anneau (3) qui est soumis à l'action d'un ressort de compression (4) pour per mettre un maintien étanche du sachet hygiénique (1) entre la butée (2) et l'anneau (3), un robinet (5) de contrôle de l'écoulement de l'eau qui est maintenu fermé au repos par l'action d'un ressort de torsion (6) sur un levier (7), un passage d'eau (8), une sortie D'eau (9), un filetage (10) pour le raccordement d'un tuyau flexible d'alimentation en eau et un deuxième anneau ouvert (11) pour le maintien d'un tuyau flexible d'alimentation en air chaud. Durant le lavage des parties intimes, l'eau s'écoule par une sortie d'eau (12) du sachet hygiénique (1) qui est couverte avec une bande (13) qui permet d'éviter des éclaboussures. Le sachet hygiénique (1) a une partie cylindrique (14) qui permet d'orienter le flux d'air chaud de la sortie du tuyau flexible d'alimentation en air chaud vers les parties intimes durant le séchage. L'anneau (3) a une saillie externe qui maintient la partie (14) autour de cette sortie. Le dispositif sanitaire peut aussi être utilisé pour le lavage vaginal en utilisant un sachet hygiénique (15), pour la rééducation du périnée et le plaisir vaginal pour la femme en utilisant un sachet hygiénique (16).

Le robinet (5) dont la partie interne n'est pas représentée est réalisé dans une cavité du passage d'eau (8) du dispositif sanitaire selon la technique bien connue des robinets à sphère où l'obturateur est sphérique avec un passage d'eau diamétral et placé entre deux joints d'étanchéité. La rotation du levier (7) permet l'alignement du passage d'eau de l'obturateur sphérique avec le passage d'eau (8) et l'ouverture du robinet.

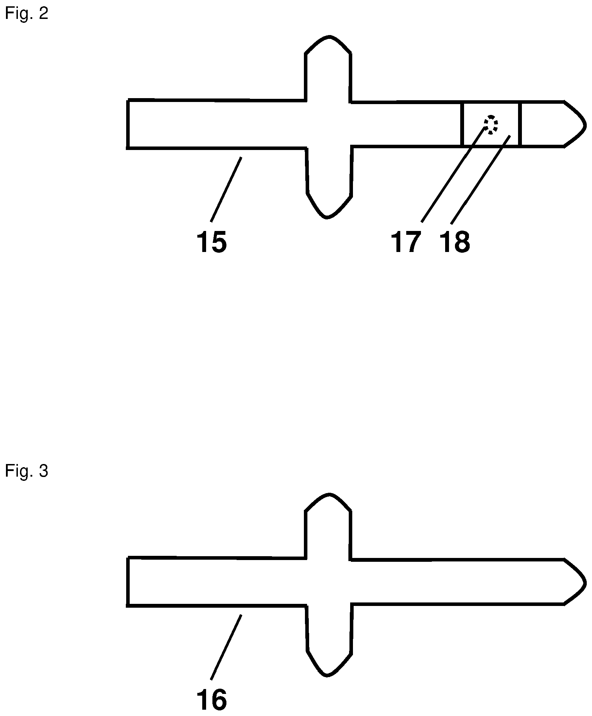

La figure 2 représente une vue de face d'un sachet hygiénique (15) destiné au lavage vaginal dont la partie centrale est plus large par rapport à celle qui est destinée à l'insertion de l'extrémité libre du dispositif sanitaire et à celle qui a la forme d'une verge et qui peut être introduite dans le vagin de l'utilisatrice. La partie centrale permet de limiter la partie qui peut être introduite dans le vagin de l'utilisatrice. Le sachet hygiénique (15) comporte une sortie d'eau (17) qui est couverte avec une bande (18) qui permet d'éviter des éclaboussures.

La figure 3 représente une vue de face d'un sachet hygiénique (16) destiné à la rééducation du périnée et au plaisir vaginal pour la femme. Il a la même forme que le sachet hygiénique (15) mais sans sortie d'eau ni bande. La partie qui a la forme d'une verge et qui peut être introduite dans le vagin de l'utilisatrice peut éventuellement être lubrifiée ou couverte avec un préservatif lubrifié avant son utilisation.

Le sachet hygiénique (15) destiné au lavage vaginal et le sachet hygiénique (16) destiné à la rééducation du périnée et au le plaisir vaginal pour la femme peuvent aussi être utilisés avec la poire de lavage qui est prévue pour des utilisations portables, un écrou et un raccord union mâle/mâle ou un tuyau souple avec un raccord mâle sur chaque extrémité. Un des filetages du raccord union peut être utilisé avec un écrou pour le maintien étanche d'un sachet hygiénique (15) ou (16) et l'autre peut être vissé sur la poire de lavage. Dans le cas d'utilisation d'un tuyau souple, un des raccords peut aussi être utilisé avec un écrou pour le maintien étanche d'un sachet hygiénique (15) ou (16) et l'autre peut être vissé sur la poire de lavage. Pour la rééducation du périnée et le plaisir vaginal pour la femme, la poire de lavage peut être remplie d'eau à environ 36°C et le sachet hygiénique doit être fixé sur cette poire de lavage au moyen d'un raccord union et d'un écrou ou d'un tuyau souple avec un raccord sur chaque extrémité et d'un écrou. Ensuite, la partie qui a la forme d'une verge du sachet hygiénique (16) doit être introduite dans le vagin de l'utilisatrice. A chaque compression de la poire de lavage, le sachet hygiénique (16) se remplit d'eau ou d'air et se tend. Il peut provoquer un plaisir vaginal pour l'utilisatrice. Dans le cas de la rééducation du périnée, l'utilisatrice doit presser alternativement la poire de lavage avec sa main et le sachet hygiénique (16) avec la contraction des muscles de son périnée en accompagnant le retour de l'eau vers la poire de lavage.

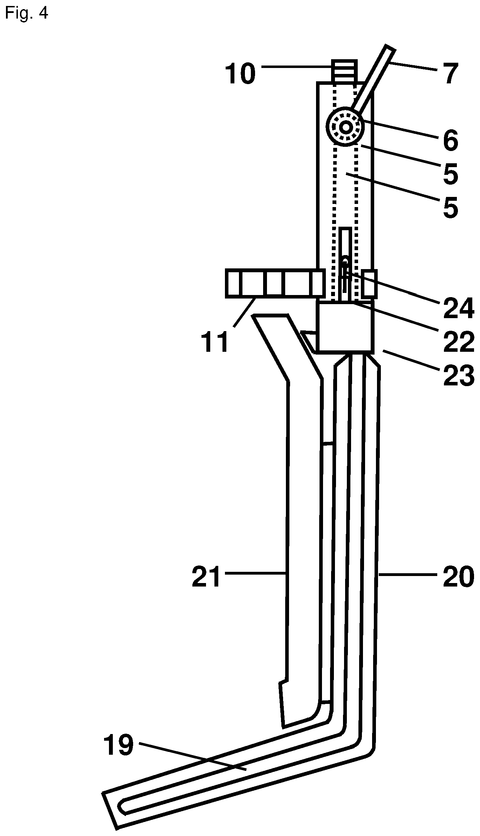

La figure 4 représente un dispositif sanitaire destiné au lavage superficiel des parties intimes par de légers frottements. Il comprend une extrémité lisse (19) qui est couverte d'un sachet hygiénique (20) transparent qui a une partie cylindrique (21) qui permet d'orienter le flux d'air chaud vers les parties intimes durant le séchage et une sortie d'eau (22) qui permet un écoulement d'eau sur ce sachet. Une pince (23) qui est maintenue fermée par un anneau élastique fendu (24) maintient le sachet hygiénique (20) et remplace la butée (2), l'anneau (3) et le ressort de compression (4). Le reste du dispositif est réalisé de façon identique à celui qui est représenté sur la figure 1.

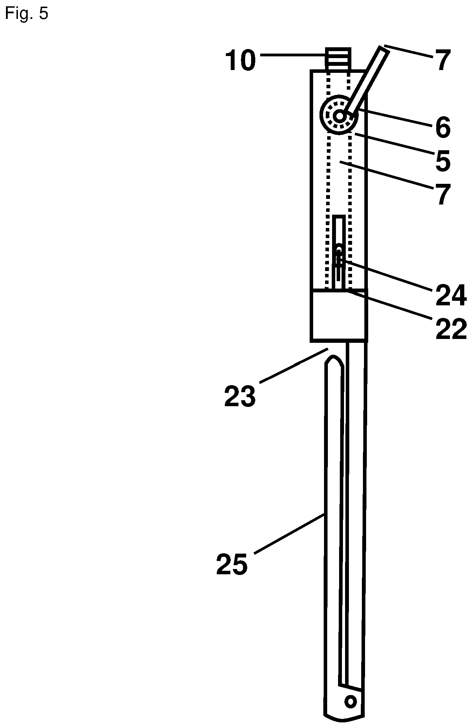

La figure 5 représente un dispositif sanitaire avec une extrémité lisse pliable (25) et sans anneau pour le maintien d'un éventuel tuyau flexible d'alimentation en air chaud. Le reste du dispositif est réalisé de façon identique à celui qui est représenté sur la figure 4.

Pour permettre l'ouverture automatique du circuit d'alimentation électrique du séchoir après le temps préfixé sur le temporisateur, le contact normalement fermé du temporisateur est connecté entre un conducteur d'alimentation électrique du séchoir et le contact normalement ouvert du contact inverseur du pressostat.

Une borne d'alimentation du temporisateur est connectée au contact normalement ouvert du contact inverseur du pressostat et l'autre borne est connectée à une borne de connexion commune à deux conducteurs d'alimentation électrique : un du séchoir et l'autre du chauffe-eau instantané.

Le deuxième conducteur d'alimentation électrique du chauffe-eau instantané est connecté au contact normalement fermé du contact inverseur du pressostat.

La phase du réseau d'alimentation électrique doit être connectée au contact commun du contact inverseur du pressostat et le neutre doit être connecté à la borne de connexion commune aux conducteurs d'alimentation électrique : du séchoir, du chauffe-eau instantané et du temporisateur.

Le pressostat est vissé sur un raccord en T pour permettre le raccordement des autres composants hydrauliques du chauffe-eau instantané. L'ordre de raccordement de ces composants n'influe pas considérablement sur le fonctionnement du chauffe-eau et séchoir.

A l'ouverture du robinet (5), la pression de l'eau dans le pressostat diminue et le contact inverseur commute la phase du réseau d'alimentation électrique du séchoir et du temporisateur qui sont connectés en parallèle au chauffe-eau instantané pour permettre le chauffage de l'eau. A la fermeture du robinet (5), la pression de l'eau dans le pressostat revient à sa valeur initiale et le contact inverseur revient aussi à sa position initiale en commutant l'alimentation électrique du chauffe-eau instantané au séchoir et au temporisateur. La durée de fonctionnement du séchoir correspond au temps préfixé sur le temporisateur.

Pour réduire au minimum le temps nécessaire au séchage, les parties latérales du sachet hygiénique (1) rempli d'eau peuvent être utilisées pour essuyer les parties intimes après chaque lavage. Dans le cas d'utilisation d'un dispositif sanitaire avec une extrémité lisse (19), les parties intimes peuvent être essuyées avec cette extrémité (19) couverte d'un sachet hygiénique (20) après chaque lavage. Les sachets hygiéniques (1,15,16,20) sont fabriqués à partir d'un matériau imperméable, essentiellement biodégradable et compostable.

Le dispositif sanitaire selon l'invention est particulièrement destiné au lavage superficiel des parties intimes, au lavage vaginal, à la rééducation du périnée et au plaisir vaginal pour la femme en utilisant le sachet hygiénique prévu pour chaque usage.

## Revendications

1. Dispositif sanitaire à main ainsi qu'un sachet hygiénique pour le lavage et le séchage des parties intimes, **caractérisé en ce que** le dispositif sanitaire comprend une butée conique (2) avec un premier anneau (3) qui est soumis à l'action d'un ressort de compression (4) pour permettre un maintien étanche d'un sachet hygiénique (1) qui se remplit d'eau pour être utilisé pour le frottement des parties intimes durant le lavage et qui a une partie cylindrique (14) qui permet d'orienter le flux d'air chaud vers les parties intimes durant le séchage, un robinet (5) de contrôle de l'écoulement de l'eau, un passage d'eau (8), une sortie d'eau (9), un filetage (10) pour le raccordement d'un tuyau flexible d'alimentation en eau et un deuxième anneau (11), étant ouvert, pour le maintien d'un tuyau flexible d'alimentation en air chaud.

2. Dispositif sanitaire à main ainsi qu'un sachet hygiénique pour le lavage et le séchage des parties intimes, **caractérisé en ce que** le dispositif sanitaire comprend une butée conique (2) avec un premier anneau (3) qui est soumis à l'action d'un ressort de compression (4) pour permettre un maintien étanche d'un sachet hygiénique (1) qui se remplit d'eau pour être utilisé pour le frottement des parties intimes durant le lavage et qui a une partie cylindrique (14) qui permet d'orienter le flux d'air chaud vers les parties intimes durant le séchage, un robinet (5) de contrôle de l'écoulement de l'eau, un passage d'eau (8), une sortie d'eau (9), un filetage (10) pour le raccordement d'un tuyau flexible d'alimentation en eau et un deuxième anneau (11), étant ouvert, pour le maintien d'un tuyau flexible d'alimentation en air chaud,
le dispositif comprenant une extrémité lisse (19) qui peut être utilisé pour le frottement superficiel des parties intimes durant le lavage en la couvrant avec un sachet hygiénique (20) qui a une partie cylindrique (21) qui permet d'orienter le flux d'air chaud vers les parties intimes durant le séchage, une sortie d'eau (22) et une pince (23) qui est maintenue fermée par un anneau élastique fendu (24) et qui remplace la butée conique (2), le premier anneau (3) et le ressort (4).

3. Dispositif sanitaire à main ainsi qu'un sachet hygiénique pour le lavage et le séchage des parties intimes, **caractérisé en ce que** le dispositif sanitaire comprend une butée conique (2) avec un premier anneau (3) qui est soumis à l'action d'un ressort de compression (4) pour permettre un maintien étanche d'un sachet hygiénique (1) qui se remplit d'eau pour être utilisé pour le frottement des parties intimes durant le lavage et qui a une partie cylindrique (14) qui permet d'orienter le flux d'air chaud vers les parties intimes durant le séchage, un robinet (5) de contrôle de l'écoulement de l'eau, un passage d'eau (8), une sortie d'eau (9), un filetage (10) pour le raccordement d'un tuyau flexible d'alimentation en eau et un deuxième anneau (11), étant ouvert, pour le maintien d'un tuyau flexible d'alimentation en air chaud,
le dispositif comprenant une extrémité lisse (19) qui peut être utilisé pour le frottement superficiel des parties intimes durant le lavage en la couvrant avec un sachet hygiénique (20) qui a une partie cylindrique (21) qui permet d'orienter le flux d'air chaud vers les parties intimes durant le séchage, une sortie d'eau (22) et une pince (23) qui est maintenue fermée par un anneau élastique fendu (24) et qui remplace la butée conique (2), le premier anneau (3) et le ressort (4),
le dispositif comprenant une extrémité lisse (25) pliable qui facilite son transport pour son utilisation avec un récipient d'eau qui est prévu pour des utilisations portables, le dispositif étant sans deuxième anneau (11).

4. Dispositif sanitaire selon l'une des revendications 1 ou 2, **caractérisé en ce qu'**il peut être alimenté en eau et en air chauds par un chauffe-eau instantané et séchoir qui comprend un pressostat avec un contact électrique inverseur qui permet la commutation de l'alimentation électrique entre le chauffe-eau et le séchoir en ouvrant ou en fermant le robinet (5), un réducteur de pression qui permet de limiter la pression de l'eau dans le tuyau flexible d'alimentation en eau et dans le sachet hygiénique qui se remplit d'eau durant le lavage des parties intimes et un temporisateur qui permet de fixer la durée de séchage à l'air chaud des parties intimes après chaque lavage.

5. Dispositif sanitaire selon l'une des revendications 1, 2 ou 3, **caractérisé en ce que** le robinet (5) de contrôle de l'écoulement de l'eau est maintenu fermé au repos par l'action d'un ressort de torsion (6) sur le levier (7).

6. Dispositif sanitaire selon la revendication 1, **caractérisé en ce qu'**un sachet hy-giènique (15) destiné au lavage vaginal a la partie centrale plus large et une partie qui a la forme d'une verge qui peut éventuellement être couverte avec un préservatif avant son utilisation.

7. Dispositif sanitaire selon les revendications 1, 2 et 6, **caractérisé en ce que** les sachets hygiéniques (1,15,20) sont fabriqués à partir d'un matériau imperméable, essentiellement biodégradable et compostable.

8. Dispositif sanitaire selon la revendication 7, **caractérisé en ce que** les sachets hygiéniques (1,15) ont des sorties d'eau respectives (12,17) qui sont couvertes avec des bandes respectives (13,18) qui permettent d'éviter des éclaboussures.

9. Dispositif sanitaire selon l'une des revendications 6, 7 ou 8, **caractérisé en ce qu'**un sachet hygiénique (16) destiné à la rééducation du périnée et le sachet hygiénique (15) peuvent être utilisés avec une poire de lavage avec filetages prévue pour des utilisations portables.

## Patentansprüche

1. Handgehaltene Hygienevorrichtung samt einem Hygienebeutel zum Waschen und Trocknen der Intimbereiche, **dadurch gekennzeichnet, dass** die Sanitäreinrichtung einen konischen Anschlag (2) mit einem ersten Ring (3) umfasst, der der Wirkung einer Druckfeder (4) ausgesetzt ist, um einen dichten Halt des Hygienebeutels (1) zu ermöglichen, der sich mit Wasser füllt, um zum Reiben der Intimbereiche während des Waschens verwendet zu werden, und der einen zylindrischen Teil (14) aufweist, der es ermöglicht, den Warmluftstrom während des Trocknens zu den Intimbereichen zu lenken, ferner umfassend einen Hahn (5) zum Regeln des Wasserflusses, einen Wasserkanal (8), einen Wasserauslass (9), ein Gewinde (10) zum Anschluss eines flexiblen Wasserversorgungsschlauchs und einen zweiten Ring (11), der offen ist, zur Aufnahme eines flexiblen Schlauchs für die Zufuhr von warmer Luft.

2. Handgehaltene Hygienevorrichtung samt einem Hygienebeutel zum Waschen und Trocknen der Intimbereiche, **dadurch gekennzeichnet, dass** die Sanitäreinrichtung einen konischen Anschlag (2) mit einem ersten Ring (3) umfasst, der der Wirkung einer Druckfeder (4) ausgesetzt ist, um einen dichten Halt des Hygienebeutels (1) zu ermöglichen, der sich mit Wasser füllt, um zum Reiben der Intimbereiche während des Waschens verwendet zu werden, und der einen zylindrischen Teil (14) aufweist, der es ermöglicht, den Warmluftstrom während des Trocknens zu den Intimbereichen zu lenken, ferner umfassend einen Hahn (5) zum Regeln des Wasserflusses, einen Wasserkanal (8), einen Wasserauslass (9), ein Gewinde (10) zum Anschluss eines flexiblen Wasserversorgungsschlauchs und einen zweiten Ring (11), der offen ist, zur Aufnahme eines flexiblen Schlauchs für die Zufuhr von warmer Luft,
wobei die Vorrichtung zusätzlich ein glattes Ende (19) aufweist, das zum sanften Reiben der Intimbereiche während des Waschens verwendbar ist, indem es mit einem Hygienebeutel (20) abgedeckt wird, der einen zylindrischen Abschnitt (21) aufweist, der das Lenken des Warmluftstroms während des Trocknens auf die Intimbereiche ermöglicht, sowie einen Wasserauslass (22) und eine Klemme (23), die durch einen geschlitzten elastischen Ring (24) geschlossen gehalten wird und die die Funktion des konischen Anschlags (2), des ersten Rings (3) und der Druckfeder (4) ersetzt.

3. Handgehaltene Hygienevorrichtung samt einem Hygienebeutel zum Waschen und Trocknen der Intimbereiche, **dadurch gekennzeichnet, dass** die Sanitäreinrichtung einen konischen Anschlag (2) mit einem ersten Ring (3) umfasst, der der Wirkung einer Druckfeder (4) ausgesetzt ist, um einen dichten Halt des Hygienebeutels (1) zu ermöglichen, der sich mit Wasser füllt, um zum Reiben der Intimbereiche während des Waschens verwendet zu werden, und der einen zylindrischen Teil (14) aufweist, der es ermöglicht, den Warmluftstrom während des Trocknens zu den Intimbereichen zu lenken, ferner umfassend einen Hahn (5) zum Regeln des Wasserflusses, einen Wasserkanal (8), einen Wasserauslass (9), ein Gewinde (10) zum Anschluss eines flexiblen Wasserversorgungsschlauchs und einen zweiten Ring (11), der offen ist, zur Aufnahme eines flexiblen Schlauchs für die Zufuhr von warmer Luft,
wobei die Vorrichtung zusätzlich ein glattes Ende (19) aufweist, das zum sanften Reiben der Intimbereiche während des Waschens verwendbar ist, indem es mit einem Hygienebeutel (20) abgedeckt wird, der einen zylindrischen Abschnitt (21) aufweist, der das Lenken des Warmluftstroms während des Trocknens auf die Intimbereiche ermöglicht, sowie einen Wasserauslass (22) und eine Klemme (23), die durch einen geschlitzten elastischen Ring (24) geschlossen gehalten wird und die die Funktion des konischen Anschlags (2), des ersten Rings (3) und der Druckfeder (4) ersetzt,
wobei die Vorrichtung ein glattes, klappbares Endstück (25) aufweist, das den Transport für die Verwendung mit einem Wasserbehälter erleichtert, der für den mobilen Einsatz vorgesehen ist, wobei die Vorrichtung in dieser Ausführungsform ohne zweiten Ring (11) ausgestattet ist.

4. Hygienevorrichtung nach einem der Ansprüche 1 oder 2, **dadurch gekennzeichnet, dass** sie mit Warmwasser und Warmluft durch einen Durchlauferhitzer und einen Trockner versorgt werden kann, wobei diese einen Pressostaten mit einem elektrischen Umschaltkontakt umfassen, der das Umschalten der Stromversorgung zwischen dem Durchlauferhitzer und dem Trockner durch das Öffnen oder Schließen des Hahns (5) ermöglicht, ferner umfassend einen Druckminderer, der den Wasserdruck im flexiblen Wasserversorgungsschlauch und im Hygienebeutel begrenzt, der sich während des Waschens der Intimbereiche mit Wasser füllt, sowie einen Zeitgeber, der die Einstellung der Dauer des Warmlufttrocknens der Intimbereiche nach jedem Waschen ermöglicht.

5. Hygienevorrichtung nach einem der Ansprüche 1, 2 oder 3, **dadurch gekennzeichnet, dass** der Hahn (5) zur Regelung des Wasserflusses durch die Wirkung einer Torsionsfeder (6) auf den Hebel (7) in Ruhestellung geschlossen gehalten wird.

6. Hygienevorrichtung nach Anspruch 1, **dadurch gekennzeichnet, dass** ein Hygienebeutel (15) zur Vaginalreinigung einen breiteren Mittelteil und einen Teil in Penisform aufweist, der vor seiner Verwendung gegebenenfalls mit einem Kondom überzogen werden kann.

7. Hygienevorrichtung nach einem der Ansprüche 1, 2 oder 6, **dadurch gekennzeichnet, dass** die Hygienebeutel (1, 15, 20) aus einem undurchlässigen, in seiner Beschaffenheit biologisch abbaubaren und kompostierbaren Material gefertigt sind.

8. Hygienevorrichtung nach Anspruch 7, **dadurch gekennzeichnet, dass** die Hygienebeutel (1, 15) jeweils über einen Wasserauslass (12, 17) verfügen, der mit einer jeweiligen Abdecklasche (13, 18) versehen ist, die dazu dient, Spritzwasser zu vermeiden.

9. Hygienevorrichtung nach einem der Ansprüche 6, 7 oder 8, **dadurch gekennzeichnet, dass** der zur Rehabilitation des Beckenbodens ausgelegte Hygienebeutel (16) sowie der Hygienebeutel (15) mit einer für den mobilen Einsatz vorgesehenen Waschbirne mit Gewinde verwendet werden können.

## Claims

1. Hand-held sanitary device and personal-hygiene sachet for washing and drying intimate body parts, **characterized in that** the hand-held sanatiry device incluses a conical stop (2) with a first ring (3) that is subject to an action of compression spring (4) to allow a tight holding of the personal-hygiene sachet (1) that is filled with water so as to be used for rubbing intimate body parts during washing and includes a cylindrical part(14) that allows a flow of hot air to be directed toward the intimate body parts during drying, a water flow control valve (5), a water passage (8), a water outlet (9), a thread (10) for coupling a water supply hose and a second ring (11), being open, for holding a hot air supply hose.

2. Hand-held sanitary device and personal-hygiene sachet for washing and drying intimate body parts, **characterized in that** the hand-held sanatiry device incluses a conical stop (2) with a first ring (3) that is subject to an action of compression spring (4) to allow a tight holding of the personal-hygiene sachet (1) that is filled with water so as to be used for rubbing intimate body parts during washing and includes a cylindrical part(14) that allows a flow of hot air to be directed toward the intimate body parts during drying, a water flow control valve (5), a water passage (8), a water outlet (9), a thread (10) for coupling a water supply hose and a second ring (11), being open, for holding a hot air supply hose,
the device including a smooth end (19) configured for a surface rubbing of the intimate body parts during washing with covering the smooth end (19) with a personal-hygiene sachet (20) that has a cynlidrical part (21) that allows to direct the flow of hot air toward the intimate body parts during drying, a water outlet (22) and a clip (23) that is kept closed by a split elastic ring (24) and that replaces the conical stop (2), the first ring (3) and the spring (4).

3. Hand-held sanitary device and personal-hygiene sachet for washing and drying intimate body parts, **characterized in that** the hand-held sanatiry device incluses a conical stop (2) with a first ring (3) that is subject to an action of compression spring (4) to allow a tight holding of the personal-hygiene sachet (1) that is filled with water so as to be used for rubbing intimate body parts during washing and includes a cylindrical part(14) that allows a flow of hot air to be directed toward the intimate body parts during drying, a water flow control valve (5), a water passage (8), a water outlet (9), a thread (10) for coupling a water supply hose and a second ring (11), being open, for holding a hot air supply hose,
the device including a smooth end (19) configured for a surface rubbing of the intimate body parts during washing with covering the smooth end (19) with a personal-hygiene sachet (20) that has a cynlidrical part (21) that allows to direct the flow of hot air toward the intimate body parts during drying, a water outlet (22) and a clip (23) that is kept closed by a split elastic ring (24) and that replaces the conical stop (2), the first ring (3) and the spring (4),
the device including a foldable smooth end (25) which facilitates the tranport of the hand-held sanitary device for use with a water contenair which is intended for portable uses, the device being without the second ring (11).

4. Sanitary device according to one of the claims 1 or 2, **characterized in that** it can be supplied with hot water and hot air by an instantaneous water heater and dryer which includes a pressure switch with an electric change-over contact that switches a power supply between the instantaneous water heater and the dryer by opening or closing the water flow control valve (5), a pressure reducer which limits a water pressure in the water supply hose and in the personal-hygiene sachet wich fills up with water when washing the intimate boby parts and a timer which allows to set a duration of hot air drying of the intimate body parts after each wash.

5. Sanitary device according to one of the claims 1, 2 or 3, **characterized in that** the water flow control valve (5) is kept closed at rest by action of a torsion spring (6) on a lever (7).

6. Sanitary device according to the claim 1, **characterized in that** a personal-hygiene sachet (15) intended for vaginal washing has a central part that is wider in a center and a part that is shaped like a penis that may be covered with a condom prior to its use.

7. Sanitary device according to the claims 1, 2 and 6, **characterized in that** the personal-hygiene sachets (1,15,20) are made of an impermeable, essentially biodegratable and compostable material.

8. Sanitary device according to the claim 7, **characterized in that** the personal-hygiene sachets (1,15) have respective water outlet (12,17) that are covered with respective stips (13,18) wich prevent spashing.

9. Sanitary device according to one of the claims 6, 7 or 8, **characterized in that** a personal-hygiene sachet (16) intended for perineal reeducation and the personal-hygiene sachets (15) can be used with a washing bulb with threads provided for portable uses.
